(19) Europäisches Patentamt
European Patent Office
Office européen des brevets

(11) Publication number: 0 172 917 A1

(12) # EUROPEAN PATENT APPLICATION

published in accordance with Art. 158(3) EPC

(21) Application number: 85901075.3

(51) Int. Cl.⁴: A 61 K 31/435

(22) Date of filing: 25.02.85

Data of the international application taken as a basis:

(86) International application number: PCT/JP 85/00085

(87) International publication number: WO 85/03866 (12.09.85 85/20)

(30) Priority: 02.03.84 JP 40836/84

(43) Date of publication of application: 05.03.86 Bulletin 86/10

(84) Designated Contracting States: BE CH DE FR GB LI NL SE

(71) Applicant: **Yoshitomi Pharmaceutical Industries, Ltd.,** 35 Hiranomachi 3-chome Higashi-ku, Osaka-shi Osaka 541 (JP)

(72) Inventor: **HISATOME, Masao**, 839-15, Wakamatsucho, Tokorozawa-shi Saitama 359 (JP)
Inventor: **TERASAWA, Michio**, 5-18, Chuomachi 2-chome, Nakatsu-shi Oita 871 (JP)
Inventor: **YASUDA, Hiroshi**, 5-16, Chuomachi 2-chome, Nakatsu-shi Oita 871 (JP)

(74) Representative: von Kreisler, Alek, Dipl.-Chem. et al, Deichmannhaus am Hauptbahnhof, D-5000 Köln 1 (DE)

(54) **AGENTS FOR TREATING IMMUNODEFICIENCY, METHOD FOR TREATING THEM, AND USE THEREOF.**

(57) Use and method for preventing and treating immunodeficiency using 8-(2-dimethylaminoethyl)-3-oxo-4-phenyl-1-thia-4,8-diazaspiro[4,5]decane represented by formula (I), or a pharmaceutically acceptable acid addition salt and/or hydrate thereof.

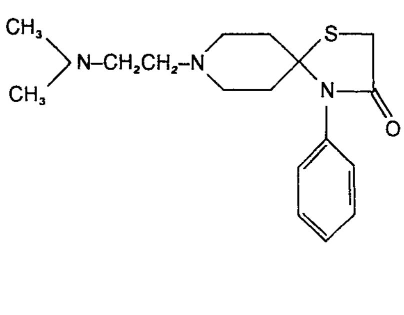

(I)

EP 0 172 917 A1

SPECIFICATION

Drugs for diseases caused by immunoabnormality, methods of the treatment and uses

## Field of the Invention

The present invention relates to a pharmaceutical field, and, in more detail, to a use of a known compound for the manufacture of a novel drug for the prophylaxis and treatment of diseases associated with immunoabnormality and a method of the prophylaxis and treatment of such diseases.

## Background of the Invention

Recently, diseases caused by disorder of immune function such as autoimmune diseases, infectious diseases or immune depressant diseases are gradually increased in human beings.

It is known that adrenocortical hormones cause severe adverse effects such as immunoabnormality, infectious diseases or edema, although they exhibit drastic effects and are one of the most widely employed drugs.

Accordingly, various immunomodulators such as levamisole or D-penicillamine are developed, but their therapeutic effects are not to be satisfied. Various investigations on the developments of effective drugs and methods of treatment have been still continued.

German Patent specification No. 2435381 discloses

8-(2-diethylaminoethyl)-3-oxo-4-phenyl-1-thia-4,8-diazaspiro-[4.5]decane of the formula:

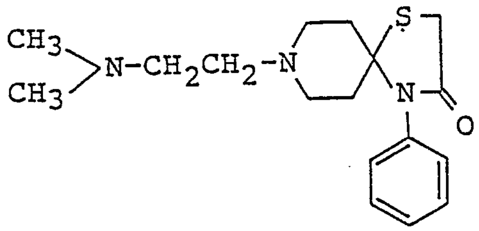

(I)

or an acid addition salt thereof which show potent effects on experimental liver dysfunctions.

## Disclosure of the Invention

The present inventors have made intensive studies in order to develop potent and new drugs for the prophylaxis and treatment of various diseases associated with immunoabnormality. As a result of such investigations, the present inventors have found that the compound of formula (I) mentioned above or a pharmaceutically acceptable acid addition salt thereof and/or hydrate thereof have effective immunomodulatory activity, and completed the present invention.

The present invention relates to a use of the compound of formula (I) or a pharmaceutically acceptable acid addition salt thereof and/or hydrate thereof for the manufacture of a drug for the prophylaxis and treatment of diseases associated with immunoabnormality, and to a method of the prophylaxis and treatment of diseases associated with immunoabnormality in human beings by administering a therapeutically affective amount of the compound of formula (I) or a pharmaceutically acceptable acid addition salt thereof and/or hydrate thereof.

The pharmaceutically acceptable acid addition salt of the compound of formula (I) includes, for example, a salt with an inorganic acid such as hydrochloric acid, hydrobromic acid, sulfuric acid, nitric acid or phosphoric acid and a salt with an organic acid such as citric acid, fumaric acid, maleic acid, tartaric acid, malonic acid, pamoic acid, salicylic acid, O-(4-hydroxybenzoyl)benzoic acid, methanesulfonic acid or p-toluenesulfonic acid.

The preferable compound of the present invention is 8-(2-diethylaminoethyl)-3-oxo-4-phenyl-1-thia-4,8-diazaspiro-[4.5]decane dihydrochloride monohydrate [melting point : 277-278°C with decomposition, hereinafter referred to as Compound (I)].

The following pharmacological experiments illustrate that the compounds of the present invention have immunomodulatory activity.

Experiment 1: Stimulative effect on immune response

Six week-old female C57BL/6 mice were immunized with the intraperitoneal administration of 5 x $10^8$ sheep red blood cells. Compound (I) was administered orally for 4 days till the 3rd day after immunization. On the 4th day after immunization, the numbers of thymus-rossete forming cells (RFC) in mice were assessed by the method of Bach et al [Cellular Immunology, vol. 3, p. 1 (1972)]. Viable thymus lymphocytes which attached to four or more sheep red blood cells were determined as RFC. The results were expressed as the ratio (%) of the number of RFC to thymus lymphocytes, as shown in Table 1. In control group, 0.5% methylcellulose solution was orally administered

into mice.

Table 1

| | Dose (mg/kg) | No. of mice | Ratio (%) of RFC (mean ± standard error) | %increase |
|---|---|---|---|---|
| Control | 0 | 8 | 1.06±0.31 | - |
| Compound (I) | 3 | 8 | 5.33±0.99** | 403 |
| Compound (I) | 30 | 8 | 6.28±1.27** | 492 |

**: P<0.01 significant vs control

As shown in Table 1, Compound (I) enhanced the production of RFC.

Experiment 2: Restorative effect on reduced immune response

The same procedures as shown in Experiment 1 were performed except that six week-old female BALB/c mice were immunized with 5 x $10^8$ sheep red blood cells and 20 mg/kg cyclophosphamide was orally administered to mice the day before and on immunization. The results are shown in Table 2.

Table 2

| | Dose (mg/kg) | No. of mice | Ratio (%) of RFC (mean ± standard error) | % decrease |
|---|---|---|---|---|
| Control | 0 | 6 | 6.81±1.38 | - |
| Cyclophosphamide | 20 | 6 | 1.14±0.63 | 79 |
| Cyclophosphamide<br>Compound (I) | 20<br>10 | 6 | 1.78±0.80 | 74 |
| Cyclophosphamide<br>Compound (I) | 20<br>30 | 6 | 3.86±0.52** | 43 |
| Cyclophosphamide<br>Compound (I) | 20<br>100 | 6 | 5.26±0.64** | 23 |

**: P<0.01 significant vs cyclophosphamide group

As shown in Table 2, the thymus-RFC production was decreased by the pretreatment with cyclophosphamide by about 79%. Compound (I) at doses of 30 mg/kg or more restored the cyclophosphamide-induced suppression of RFC production. These results indicate that Compound (I) restores the suppression of RFC production in mice.

Experiment 3: Stimulative effect on phagocytosis of leukocytes

Effect of Compound (I) on phagocytosis was examined using rat peritoneal leukocytes according to the method of Stossel et al described in Journal of Clinical Investigation, vol. 51, p. 615 (1972). The results are shown in Table 3.

Table 3

| | Concentration (μM) | Number | % increase of phagocytosis |
|---|---|---|---|
| Compound (I) | 10 | 2 | 27.1 |
| Compound (I) | 100 | 2 | 61.4 |

Table 3 indicates that Compound (I) enhanced phagocytosis at concentrations of 10 μM or more.

Experiment 4: Stimulative effect on phagocytosis of macrophages

Casein sodium was injected intraperitoneally into rats. Three days later, peritoneal macrophages were harvested. Phagocytosis was assessed by the same procedures as shown in Experiment 3. The results are shown in Table 4.

Table 4

| | Concentration (μM) | Number | % increase of phagocytosis |
|---|---|---|---|
| Compound (I) | 10 | 2 | 12.5 |
| Compound (I) | 100 | 2 | 30.6 |

As shown in Table 4, Compound (I) also enhanced phagocytosis of macrophages, as did that of leukocytes. This property indicates that Compound (I) enhances the functions of reticuloendothelial system in vivo.

Experiment 5: Stimulative effect on cell-mediated immunity

Eight week-old female C57BL/6 mice were injected subcutaneously in the back with 0.1 ml of a 0.25% methylated human serum albumin (hereinafter referred to as MeHSA). Compound (I) was administered orally for 4 consecutive days from the day of the sensitization. One hour after the last administration of Compound (I), 0.025 ml of a 0.1% MeHSA solution was injected subcutaneously on the left hindpaw. The thickness of footpad was measured the 24 hours after the injection using a dissecting microscope by the method of Baba et al [Acta Pathology Japan, vol. 27, p. 165 (1977)]. The difference between the thickness of footpad before and after the injection of MeHSA was expressed as the delayed type hypersensitivity (hereinafter referred to as DTH). The results of this experiment are shown in Table 5.

Table 5

| | Dose (mg/kg) | No. of mice | DTH (mm) (mean ± standard error) | % increase |
|---|---|---|---|---|
| Control | 0 | 10 | 0.21±0.02 | - |
| Compound (I) | 10 | 10 | 0.22±0.02 | 5 |
| Compound (I) | 100 | 10 | 0.42±0.03** | 100 |
| Compound (I) | 300 | 10 | 0.33±0.03** | 57 |

**: P<0.01 significant vs control

Since Table 5 shows that Compound (I) enhanced the

MeHSA-induced DTH, an index of the cell-mediated immune response, it can be understood that Compound (I) has an immunostimulative activity on cell-mediated immunity.

Experiment 6: Stimulative effect on reduced cell-mediated immunity

To 8 week-old male spontaneous hypertensive rats (hereinafter referred to as SHR), cell-mediated immunodysfunction animals, was orally administered Compound (I) for 20 consecutive days. Twenty-four hours after the last administration, thymus was removed and cell suspension was prepared. The numbers of spontaneous rosette forming cells (hereinafter referred to as S-RFC) were measured the method of Takeichi and Boone [Cellular Immunology, vol. 27, p. 52 (1976)]. The results are expressed as the percentage of rosette forming cells in total thymus cells and shown in Table 6.

Table 6

| | Dose (mg/kg) | No. of mice | Ratio (%) of S-RFC (mean ± standard error) | % increase |
|---|---|---|---|---|
| Control | 0 | 10 | 46.7±2.9 | - |
| Compound (I) | 25 | 10 | 55.0±5.2 | 18 |
| Compound (I) | 100 | 10 | 57.8±3.6** | 24 |

**: P<0.01 significant vs control

Compound (I) enhanced S-RFC in SHR with depressed S-RFC and exhibited stimulative activity on the function of thymus cells.

Experiment 7: Immunosuppressive effect

Effect of Compound (I) on the MeHSA-induced DTH was examined in 10 week-old female BALB/c mice, higher responder strain to

the MeHSA-induced DTH, by the same procedures as described in Experiment 5. The results are shown in Table 7.

Table 7

| | Dose (mg/kg) | No. of mice | DTH (mm) (mean ± standard error) | % inhibition |
|---|---|---|---|---|
| Control | 0 | 10 | 0.96±0.09 | - |
| Compound (I) | 3 | 10 | 0.91±0.04 | 5 |
| Compound (I) | 30 | 10 | 0.77±0.03* | 20 |
| Compound (I) | 300 | 10 | 0.71±0.04** | 36 |

*: $P<0.05$, **: $P<0.01$ significant vs control

Table 7 shows that Compound (I) inhibited the high DTH response in BALB/c mice, and Compound (I) has immunosuppressive activity on the high cell-mediated immunity.

Experiment 8: Inhibitory effect on the production of autoantibody

To 4 week-old female NZB/W $F_1$ mice was orally administered Compound (I) three times every week and, at 7 week-old mice blood was gathered from the eyegrounds and the serum was separated. The detection of natural thymotoxic autoantibody (hereinafter referred to as NTA) was performed by the method of Takeichi et al [Cellular Immunology, vol. 60, p. 181 (1981)]. The results are expressed as the percentage of dead cells in thymus cells and shown in Table 8.

Table 8

| | Dose (mg/kg) | No. of mice | Dilution of serum | NTA (%) (mean ± standard error) |
|---|---|---|---|---|
| Control | 0 | 10 | 64 | 28.0±0.8 |
| Compound (I) | 30 | 10 | 64 | 9.5±0.6** |

Table 8 (continued)

| | Dose (mg/kg) | No. of mice | Dilution of serum | NTA (%) (mean ± standard error) |
|---|---|---|---|---|
| Control | 0 | 10 | 128 | 20.3±1.1 |
| Compound (I) | 30 | 10 | 128 | 8.7±0.5** |

**: $P<0.01$ significant vs control

As shown in Table 8, Compound (I) inhibited the production of autoantibody in NZB/W $F_1$ mice. Therefore, it indicates that Compound (I) inhibits the production of various autoantibodies.

Experiment 9: Acute toxicity

Compound (I) was administered orally or intraperitoneally to groups of 8 male Wistar rats weighing about 200 g. After one week, acute toxicity ($LD_{50}$ value) was determined by Probit method. The results are shown in Table 9.

Table 9

| Route of administration | $LD_{50}$ |
|---|---|
| oral | >8,000 mg/kg |
| intraperitoneal | 827 mg/kg |

$LD_{50}$ values of Compound (I) in groups of 10 male dd-strain mice weighing 20-25 g were also determined. The results are shown in Table 10.

Table 10

| Route of administration | $LD_{50}$ |
|---|---|
| oral | 1,841 mg/kg |
| intraperitoneal | 474 mg/kg |

No sex difference in $LD_{50}$ values was observed in both

animals.

It is apparent from the above results of various experiments that the compounds of the present invention have potent stimulative effect on low immune response, restorative effect on reduced immune response, stimulative effect on phagocytosis of leukocytes and macrophages, and inhibitoty effect on high immune response and the production of autoantibody, indicating that the compounds of the present invention as an active ingredient enhance the function of reticuloendothelial system and improve the immune response. Furthermore, the compounds of the present invention have very low toxicity.

Therefore, the compounds of the present invention can be effectively and safely administered to human beings for the prophylaxis and treatment of various diseases associated with immunoabnormality in human beings.

The diseases associated with immunoabnormality which can be prevented or treated with the compounds of the present invention include, for example, allergic diseases, autoimmune diseases such as erythematosus or rheumatoid arthritis and immune depressive diseases such as infectious diseases or other diseases caused by cancer or surgery.

The drugs of the present invention can be preferably manufactured by admixing the compounds of the present invention as an active ingredient with a conventional and pharmaceutically acceptable additives such as carrier, excipient or diluent, and administered orally or parenterally in the form of tablets, granules, powder, capsules, syrup, injectable solutions or external preparations. The doses are sufficient to a therapeutically effective amount of the compounds of the present

invention, and may vary depending upon symptoms, the responses to drugs, the forms of administration or age and so on. The daily dose for human adults usually ranges from about 10 to 600 mg in a single or multiple dose.

Pharmaceutical composition 1: 50 mg Tablets

To 50 g of pulverized Compound (I) are added 70 g of lactose, 25 g of microcrystalline cellulose, 19.5 g of corn starch, 5 g of talc and 0.5 g of magnesium stearate and mixed well to compress into tablets weighing 170 mg.

Pharmaceutical composition 2: 50 mg Film-coated tablets

To 50 g of Compound (I) are added 25 g of lactose, 17 g of starch, 10 g of microcrystalline cellulose, 7.5 g of talc and 0.5 g of magnesium stearate and mixed well to compress into tablets weighing 110 mg.

The tablets are coated with a mixture of hydroxypropylmethylcellulose, polyethylene glycol and talc to give film-coated tablets.

Pharmaceutical composition 3: Injectable solutions

A mixture of 10 g of Compound (I) and 8 g of sodium chloride is dissolved into distilled water for injection and the pH is adjusted to 7-8 and then the whole volume is made to 1 liter. The solution is filtered with a membrane filter with 0.22 μ mesh and each 2 ml of the solution is filled into an ampule. The ampule is subjected to steam sterilization under pressure at 120°C for 20 minutes.

Although the present invention has been adequately discussed in the foregoing specification and examples included therein, one readily recognizes that various changes and modifications may be made without departing from the spirit and scope thereof.

What is claimed is:

1. A use of 8-(2-dimethylaminoethyl)-3-oxo-4-phenyl-1-thia-4,8-diazaspiro[4,5]decane of the formula:

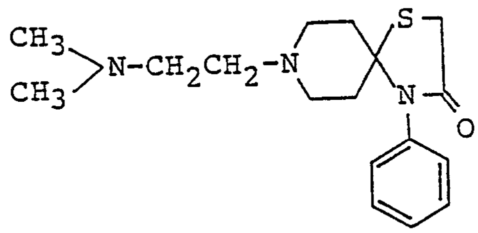

or a pharmaceutically acceptable acid addition salt thereof and/or hydrate thereof for the manufacture of a drug for the prophylaxis and treatment of diseases associated with immunoabnormality.

2. The use of claim 1 wherein the compound is 8-(2-dimethylaminoethyl)-3-oxo-4-phenyl-1-thia-4,8-diazaspiro[4.5]decane dihydrochloride monohydrate.

3. A method of the prophylaxis and treatment of diseases associated with immunoabnormality in human beings which comprises administering a therapeutically effective amount of 8-(2-dimethylaminoethyl)-3-oxo-4-phenyl-1-thia-4,8-diazaspiro[4.5]decane of the formula:

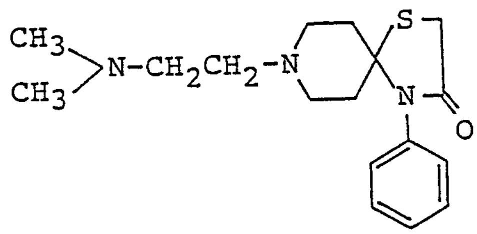

or a pharmaceutically acceptable acid addition salt thereof and/or hydrate thereof.

4. The method of claim 3 which comprises administering ..

8-(2-dimethylaminoethyl)-3-oxo-4-phenyl-1-thia-4,8-diazaspiro-[4.5]decane dihydrochloride monohydrate.

# INTERNATIONAL SEARCH REPORT

0172917

International Application No. PCT/JP85/00082

**I. CLASSIFICATION OF SUBJECT MATTER** (if several classification symbols apply, indicate all) [3]

According to International Patent Classification (IPC) or to both National Classification and IPC

Int. $Cl^4$ A61K31/435

**II. FIELDS SEARCHED**

Minimum Documentation Searched [4]

| Classification System | Classification Symbols |
|---|---|
| IPC | A61K31/435, C07D513/10, 498/10 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| A | JP, B1, 49-28196 (Yoshitomi Pharmaceutical Industries, Ltd.) 24 July 1974 (24. 07. 74), (Family nashi) | 1 - 4 |
| A | JP, B2, 51-5387 (Yoshitomi Pharmaceutical Industries, Ltd.) 19 February 1976 (19. 02. 76), (Family nashi) | 1 - 4 |
| A | DE, A, 2435381 (Yoshitomi Pharmaceutical Industries, Ltd.) 5 February 1976 (05. 02. 76), (Family nashi) | 1 - 4 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| May 14, 1985 (14. 05. 85) | May 27, 1985 (27. 05. 85) |
| **International Searching Authority [1]** | **Signature of Authorized Officer [20]** |
| Japanese Patent Office | |